# EUROPEAN PATENT APPLICATION

(11) **EP 1 430 894 A1**
(43) Date of publication of application: **23.06.2004**
(21) Application number: 02798032.5
(22) Date of filing: 05.09.2002
(51) Int. Cl.: A61K 31/404, A61K 31/416, A61K 31/4178, A61K 31/427, A61K 31/437, A61K 31/4439, A61K 45/00, A61P 1/00, A61P 1/02, A61P 3/10, A61P 5/50, A61P 19/02, A61P 19/04, A61P 25/00, A61P 27/02

(54) **LYMPHOCYTIC ACTIVATION INHIBITOR AND REMEDIAL AGENT FOR AUTOIMMUNE DISEASE**

(30) Priority: 05.09.2001 JP 2001269480
(71) Applicant: Eisai Co. Ltd, Tokyo 112-8088 (JP)
(72) Inventor: HANADA, Takahisa, Tsukuba-shi, Ibaraki 305-0003 (JP); YAMAUCHI, Toshihiko, Tsukuba-shi, Ibaraki 305-0045 (JP); CHIBA, Kenichi, Tsuchiura-shi, Ibaraki 300-0038 (JP); OWA, Takashi, Tsukuba-shi, Ibaraki 305-0861 (JP); HIDA, Takayuki, Tsukuba-shi, Ibaraki 305-0051 (JP); MIYAMOTO, Norimasa, Tsukuba-shi, Ibaraki 305-0051 (JP)
(74) Representative: HOFFMANN EITLE
(86) International application number: PCT/JP2002/009030
(87) International publication number: WO 2003/022271

(57) **Abstract**

A lymphocyte activation inhibitor and a therapeutic agent for an autoimmune disease, each comprising, as an active ingredient, a sulfonamide derivative or sulfonic acid ester derivative represented by the following general formula (I): 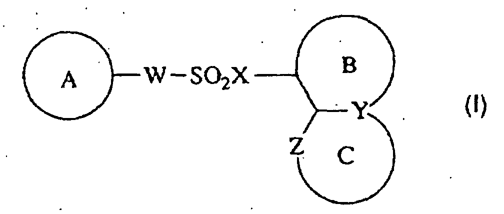 wherein the ring A represents a monocyclic or bicyclic aromatic ring which may be substituted,
the ring B represents a 6-membered unsaturated hydrocarbon ring or a 6-membered unsaturated heterocyclic ring containing one nitrogen atom as a heteroatom, each of which may be substituted,
the ring C represents a 5-membered heterocyclic ring containing one or two nitrogen atoms, which may be substituted,
W represents a single bond or -CH=CH-,
X represents -N(R¹)- or an oxygen atom,
Y represents a carbon atom or a nitrogen atom,
Z represents -N(R²)- or a nitrogen atom, and
R¹ and R² may be identical or different and each represents a hydrogen atom or a lower alkyl group, or a pharmacologically acceptable salt thereof, or a hydrate thereof.

## Description

### Technical Field

The present invention relates to a lymphocyte activation inhibitor. The present invention also relates to a therapeutic agent for an autoimmune disease, and more specifically to a therapeutic agent that is effective for rheumatism, multiple sclerosis, neuro-autoimmune diseases, type I (insulin-dependent) diabetes, systemic lupus erythematosus (SLE), inflammatory bowel diseases (IBD), and Sjogren's syndrome.

### Background Art

It has been elucidated that division and activation of autoantigen-specific lymphocytes significantly contribute to aggravation of a pathological condition in autoimmune diseases. In other words, the autoantigen-specific lymphocytes get activated and proliferate by recognizing a part of the self as a foreign body, and induce disorders in tissues by causing activation of cells in other immune systems, thereby playing a significantly important role in the development of pathological conditions. Therefore, inhibiting the lymphocyte activation induced by an antigen allows the inhibition of development of an autoimmune disease. Specific examples of the autoimmune disease include rheumatism, multiple sclerosis, neuro-autoimmune diseases (Guillain-Barre syndrome, neuro-Behcet's disease, etc.), type I (insulin-dependent) diabetes, systemic lupus erythematosus (SLE), inflammatory bowel diseases (IBD), and Sjogren's syndrome, which are known as intractable diseases [reference document: Clinical Immunology and Immunopathology, 84, 223-243 (1997)].

Currently, lymphocyte activation inhibitors based on various action mechanisms have been clinically used for treatments of the autoimmune diseases described above. However, it is known that they accompany severe side effects in some cases, though their effectiveness are recognized. As a result, they have not been used widely, and the development of a lymphocyte activation inhibitor having a lower toxicity has been desired.

JP 07-165708 A, JP 08-231505 A, and JP 2000-247949 A disclose a sulfonamide compound or a sulfonic acid ester compound, whereas those publications describe nothing about the lymphocyte activation inhibition effect thereof.

### Disclosure of the Invention

An object of the present invention is to provide a lymphocyte activation inhibitor having an excellent lymphocyte activation inhibition effect. Another object of the present invention is to provide a therapeutic agent for an autoimmune disease based on a lymphocyte activation inhibition effect.

In view of the above, the inventors of the present invention have made intensive studies for seeking an excellent lymphocyte activation inhibitor, and found that a sulfonamide compound and a sulfonic acid ester compound having a bicyclic hetero ring have an excellent lymphocyte activation inhibition effect and also have less toxicity, thus completing the present invention.

The present invention provides a lymphocyte activation inhibitor comprising, as an active ingredient, a sulfonamide derivative or sulfonic acid ester derivative represented by the following general formula (I): 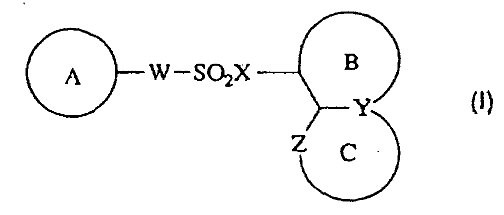 wherein the ring A represents a monocyclic or bicyclic aromatic ring which may be substituted,
the ring B represents a 6-membered unsaturated hydrocarbon ring or a 6-membered unsaturated heterocyclic ring containing one nitrogen atom as a heteroatom, each of which may be substituted,
the ring C represents a 5-membered heterocyclic ring containing one or two nitrogen atoms, which may be substituted,
W represents a single bond or -CH=CH-,
X represents -N(R¹)- or an oxygen atom,
Y represents a carbon atom or a nitrogen atom,
Z represents -N(R²)- or a nitrogen atom, and
R¹ and R² may be identical or different and each represents a hydrogen atom or a lower alkyl group, or a pharmacologically acceptable salt thereof, or a hydrate thereof.

The present invention also provides a therapeutic agent for an autoimmune disease, comprising, as an active ingredient, a sulfonamide derivative or sulfonic acid ester derivative represented by the above general formula (I), or a pharmacologically acceptable salt thereof, or a hydrate thereof.

In the general formula (I), it is preferred that W is a single bond. More preferably, X and Z are -NH-, and Y is a carbon atom.

In the general formula (I), it is preferred that the ring B is benzene or pyridine, each of which may be substituted.

In the general formula (I), it is preferred that the ring C is pyrrole which may be substituted.

In the general formula (I), it.is preferred that the ring A is benzene or pyridine, each of which may be substituted; the ring B is benzene which may be substituted; the ring C is pyrrole which may be substituted; W is a single bond; and X and Z are -NH-.

Examples of the autoimmune disease include cellular autoimmune diseases, rheumatism, multiple sclerosis, neuro-autoimmune diseases, type I (insulin-dependent) diabetes, systemic lupus erythematosus, inflammatory bowel diseases, and Sjogren's syndrome.

When the autoimmune disease is multiple sclerosis, it is preferred that the agent further comprises a drug having a neuron-protecting effect.

### Brief Description of the Drawings

Fig. 1 shows effects on mouse collagen-induced arthritis.
Fig. 2 shows effects in an experimental encephalomyelitis model.

### Best Mode for Carrying out the Invention

In the general formula (I), the "monocyclic or bicyclic aromatic ring which may be substituted" represented by the ring A is an aromatic hydrocarbon ring or an aromatic heterocyclic ring containing at least one of nitrogen, oxygen and sulfur atoms, each of which may have one to three substituents thereon. Examples of such aromatic rings included in the ring A include pyrrole, pyrazole, imidazole, thiophene, furan, thiazole, oxazole, benzene, pyridine, pyrimidine, pyrazine, pyridazine, naphthalene, quinoline, isoquinoline, phthalazine, naphthyridine, quinoxaline, quinazoline, cinnoline, indole, isoindole, indolizine, indazole, benzofuran, benzothiophene, benzoxazole, benzimidazole, benzopyrazole, benzothiazole and so forth. They may have one to three substituents. When two or more substituents are present, they may be the same or different. Examples of the substituents include an amino group which may be substituted with a lower alkyl gourp or a lower cycloalkyl group; a lower alkyl group; a lower alkoxy group; hydroxyl; nitro; mercapto; cyano; a lower alkylthio group; a halogen group; a group represented by the formula -a-b wherein a represents a single bond, -(CH₂)ₖ-, -O-(CH₂)ₖ-, -S-(CH₂)ₖ- or -N(R³)-(CH₂)ₖ-, k is an integer of 1 to 5, R³ represents a hydrogen atom or a lower alkyl group, and b represents -CH₂-d (wherein d represents an amino group which may be substituted with a lower alkyl group, a halogen group, hydroxyl, a lower alkylthio group, cyano or a lower alkoxy group); a group represented by the formula -a-e-f wherein a has the same meaning as defined above, e represents -S(O)- or -S(O)₂-, f represents an amino group which may be substituted with a lower alkyl group or a lower alkoxy group, a lower alkyl group, trifluoromethyl, -(CH₂)ₘ-b or -N(R⁴)-(CH₂)ₘ-b (wherein b has the same meaning as defined above, R⁴ represents a hydrogen atom or a lower alkyl group, and m is an integer of 1 to 5); a group represented by the formula -a-g-h wherein a has the same meaning as defined above, g represents -C(O)- or -C(S)-, h represents an amino group which may be substituted with a lower alkyl group, hydroxyl, a lower alkyl group, a lower alkoxy group, - (CH₂)ₙ-b or -N(R⁵)-(CH₂)ₙ-b (wherein b has the same meaning as defined above, R⁵ represents a hydrogen atom or a lower alkyl group, and n is an integer of 1 to 5); a group represented by the formula -a-N(R⁶)-g-i wherein a and g have the same meanings as defined above, R⁶ represents a hydrogen atom or a lower alkyl group, i represents a hydrogen atom or a lower alkoxy group or f (f has the same meaning as defined above); a group represented by the formula -a-N(R⁷)-e-f wherein a, e and f have the same meanings as defined above, and R⁷ represents a hydrogen atom or a lower alkyl group; a group represented by the formula -(CH₂)ₚ-j-(CH₂)_{q}-b wherein j represents an oxygen atom or a sulfur atom, b has the same meaning as defined above, and p and q may be the same or different and each represents an integer of 1 to 5; a group represented by the formula -(CH₂)ᵤ-Ar wherein Ar represents a phenyl group or a heteraryl group, which may be substituted with a lower alkyl group, a lower alkoxy group or a halogen atom and u represents 0 or an interger of 1 to 5; a group represented by the formula -CONH-(CH₂)ᵤ-Ar wherein Ar and u have the same meaning as defined above; a group represented by the formula -SO₂-(CH₂)ᵤ-Ar wherein Ar and u have the same meaning as defined above; and so forth.

When the substituent is an amino group substituted with two of alkyl groups, both of the alkyl groups may bond to form a 5- or 6-membered ring. Further, when the ring A is a nitrogen-containing heterocyclic ring having hydroxyl or mercapto, these groups may present in the form of an oxo or thioxo group by resonance.

The "6-membered unsaturated hydrocarbon ring or 6-membered unsaturated heterocyclic ring containing one nitrogen atom as a heteroatom, which may be substituted" represented by the ring B means benzene or pyridine which may be partially hydrogenated. It may have one or two of substituents on the ring, and when two of substituents are present, they may be the same or different.

The "5-membered heterocyclic ring containing one or two nitrogen atoms, which may be substituted" represented by the ring C means pyrrole, pyrazole or imidazole which may be partially hydrogenated. It may have one or two of substituents on the ring, and when two of substituents are present, they may be the same or different.

Examples of the substituents that the rings of B and C may have include a halogen group, cyano, a lower alkyl group, a lower alkoxy group, hydroxyl, oxo, a group represented by the formula -C(O)-r (wherein r represents a hydrogen atom, an amino group which may be substituted with a lower alkyl group, a lower alkyl group, a lower alkoxy group or hydroxyl), an amino group substituted with a lower alkyl group, trifluoromethyl and so forth.

In the general formula (I), the lower alkyl group in the definitions of R¹ and R² as well as the substituents that the rings of A, B and C may have means a linear or branched alkyl group having 1 to 6 carbon atoms, and examples thereof include methyl, ethyl, n-propyl, isopropyl, n-butyl, isobutyl, sec-butyl, tert-butyl, n-pentyl (amyl), isopentyl, neopentyl, tert-pentyl, 1-methylbutyl, 2-methylbutyl, 1,2-dimethylpropyl, n-hexyl, isohexyl, 1-methylpentyl, 2-methylpentyl, 3-methylpentyl, 1,1-dimethylbutyl, 1,2-dimethylbutyl, 2,2-dimethylbutyl, 1,3-dimethylbutyl, 2,3-dimethylbutyl, 3,3-dimethylbutyl, 1-ethylbutyl, 2-ethylbutyl, 1,1,2-trimethylpropyl, 1,2,2-trimethylpropyl, 1-ethyl-1-methylpropyl, 1-ethyl-2-methylpropyl and so forth. Among these, methyl,. ethyl, n-propyl, isopropyl, n-butyl and isobutyl are preferred, and methyl, ethyl, n-propyl and isopropyl are most preferred.

The lower cycloalkyl group mentioned in the definitions of the substituents that the ring of A may have means a cycloalkyl group having 3 to 8 carbon atoms, and examples thereof include cyclopropyl, cyclopentyl, cyclohexyl and so forth.

The lower alkoxy mentioned in the definitions of the substituents that the rings of A, B and C may have means an alkoxyl' group derived from the aforementioned lower alkyl group, such as methoxy, ethoxy, n-propoxy, isopropoxy, n-butoxy, isobutoxy and tert-butoxy. Among these, methoxy and ethoxy are most preferred. The lower alkylthio group means an alkylthio group derived from the aforementioned lower alkyl group. Further, examples of the halogen atom include a fluorine atom, a chlorine atom, a bromine atom and so forth.

The sulfonamide derivatives or the sulfonic acid ester derivatives represented by the general formula (I) may form a salt with an acid or a base. The active ingredient used in the present invention also includes salts of the sulfonamide derivatives or the sulfonic acid ester represented by the general formula (I). Examples of the salt with an acid include salts with inorganic acids, such as hydrochlorides, hydrobromides and sulfates, and salts with organic acids such as acetic acid, lactic acid, succinic acid, fumaric acid, maleic acid, citric acid, benzoic acid, methanesulfonic acid and p-toluenesulfonic acid. Examples of the salt with a base include salts with inorganic bases, such as sodium salts, potassium salts and calcium salts and salts with organic bases such as triethylamine, arginine and lysine.

It is needless to say that the compounds include hydrates and optical isomers of these compounds if they are present.

A list of specific examples of the sulfonamide derivative or the sulfonic acid ester derivative represented by the general formula (I) or the pharmacologically acceptable salt, or the hydrate thereof is as follows:
Compound 1
   N-(1H-Indol-7-yl)-4-nitrobenzenesulfonamide
Compound 2
   N-(3-Chloro-1H-indol-7-yl)-4-nitrobenzenesulfonamide
Compound 3
   4-Amino-N-(3-chloro-1H-indol-7-yl)benzenesulfonamide
Compound 4
   N-(3-Chloro-1H-indol-7-yl)-4-(methanesulfonamido)-benzenesulfonamide
Compound 5
   4-Bromomethyl-N-(1H-indol-7-yl)benzenesulfonamide
Compound 6
   N-(1,3-Dihydro-2H-indol-2-on-7-yl)-4-methylbenzenesulfonamide
Compound 7
   3-Chloro-N-(3-chloro-1H-indol-7-yl)benzenesulfonamide
Compound 8
   4-Amino-N-(3,4-dichloro-1H-indol-7-yl)benzenesulfonamide
Compound 9
   4-[N-(1H-Indol-7-yl)sulfamoyl]benzoic acid
Compound 10
   N-(3-Chloro-1H-indol-7-yl)-4-cyanobenzenesulfonamide
Compound 11
   3-Chloro-N-(3-chloro-4-methoxy-1H-indol-7-yl)-benzenesulfonamide
Compound 12
   3-Chloro-N-(3-chloro-4-hydroxy-1H-indol-7-yl)-benzenesulfonamide
Compound 13
   N-(1H-Indol-7-yl)-4-methoxybenzenesulfonamide
Compound 14
   6-Chloro-N-(3-chloro-1H-indol-7-yl)-3-pyridinesulfonamide
Compound 15
   N-(3-Chloro-1H-indol-7-yl)-4-(methylthiomethyl)-benzenesulfonamide
Compound 16
   3-Chloro-N-(3-formyl-1H-indol-7-yl)benzenesulfonamide
Compound 17
   3-Chloro-N-(3-cyano-1H-indol-7-yl)benzenesulfonamide
Compound 18
   6-Chloro-N-(3-cyano-1H-indol-7-yl)-3-pyridinesulfonamide
Compound 19
   N-(3-Chloro-1H-indol-7-yl)-4-sulfamoylbenzenesulfonamide
Compound 20
   3-Chloro-N-(8-imidazo[1,2-a]pyridinyl)benzenesulfonamide hydrochloride
Compound 21
   N-(3,4-Dichloro-1H-indol-7-yl)-4-sulfamoylbenzenesulfonamide
Compound 22
   N-(3-Chloro-1H-indol-7-yl)-4-(methylthio)benzenesulfonamide
Compound 23
   N-(3-Chloro-1H-indol-7-yl)-4-(methylsulfonyl)-benzenesulfonamide
Compound 24
   N-(3-Chloro-1H-indol-7-yl)-4-(methylsulfinyl)-benzenesulfonamide
Compound 25
   3-Chlorb-N-(3-chloro-1H-pyrrolo[3,2-c]pyridin-7-yl)-benzenesulfonamide
Compound 26
   4-Acetamido-N-(3-chloro-4-methyl-1H-indol-7-yl)-benzenesulfonamide
Compound 27
   4-Amino-N-(3-chloro-4-methyl-1H-indol-7-yl)-benzenesulfonamide
Compound 28
   4-Cyano-N-(3-chloro-1H-indol-7-yl)benzenesulfonamide
Compound 29
   4-Carbamoyl-N-(3-chloro-1H-indol-7-yl)benzenesulfonamide
Compound 30
   N-(4-Bromo-1H-indol-7-yl)-4-nitrobenzenesulfonamide
Compound 31
   N-(3-Chloro-4-cyano-1H-indol-7-yl)-4-nitrobenzenesulfonamide
Compound 32
   4-Amino-N-(3-chloro-4-cyano-1H-indol-7-yl)-benzenesulfonamide
Compound 33
   4-Amino-N-(3-chloro-1H-indol-7-yl)-3-pyridinesulfonamide
Compound 34
   N-(3-Chloro-1H-indol-7-yl)-4-(methylsulfinylmethyl)-benzenesulfonamide
Compound 35
   N-(3-Chloro-1H-indol-7-yl)-4-(2-sulfamoylethyl)-benzenesulfonamide
Compound 36
   N-(3-Chloro-1H-indol-7-yl)-4-[2-(methylsulfonyl)ethyl]-benzenesulfonamide
Compound 37
   6-Amino-N-(3-cyano-1H-indol-7-yl)-3-pyridinesulfonamide
Compound 38
   4-Acetamide-3-chloro-N-(3-chloro-1H-indol-7-yl)-benzenesulfonamide
Compound 39
   N-(3-Cyano-1H-indol-7-yl)-8-quinolinesulfonamide
Compound 40
   5-Chloro-N-(3-cyano-1H-indol-7-yl)-2-thiophenesulfonamide
Compound 41
   N-(3-Chloro-1H-indol-7-yl)-4-(methoxycarbonylamino)-benzenesulfonamide
Compound 42
   4-Acetyl-N-(3-cyano-1H-indol-7-yl)benzenesulfonamide
Compound 43
   N-(3-Chloro-1H-indol-7-yl)-4-(N-methoxysulfamoyl)-benzenesulfonamide
Compound 44
   N-(3-Cyano-1H-indol-7-yl)-β-styrenesulfonamide
Compound 45
   3-Chloro-N-(3-cyano-1H-indol-7-yl)-2-methylbenzenesulfonamide
Compound 46
   N-(3-Chloro-1H-indol-7-yl)-6-isopropylamino-3-pyridinesulfonamide
Compound 47
   N-(3-Chloro-1H-indol-7-yl)-6-[[2-(dimethylamino)-ethyl]amino]-3-pyridinesulfonamide
Compound 48
   N-(3-Cyano-1H-indol-7-yl)-2-furansulfonamide
Compound 49
   N-(3-Chloro-1H-indol-7-yl)-4-[(dimethylaminosulfonyl)-amino]benzenesulfonamide
Compound 50
   N-(3-Methyl-1H-indol-7-yl)-4-(methylsulfonyl)-benzenesulfonamide
Compound 51
   3-Cyano-N-(3-cyano-1H-indol-7-yl)benzenesulfonamide
Compound 52
   N-(3-Chloro-1H-indol-7-yl)-4-(N-methylmethanesulfonamido)-benzenesulfonamide
Compound 53
   N-(3-Chloro-1H-indol-7-yl)-4-[(methanesulfonamido)methyl]-benzenesulfonamide
Compound 54
   N-(3-Chloro-lH-indol-7-yl)-4-(1-pyrrolidinylsulfonyl)-benzenesulfonamide
Compound 55
   N-(3-Cyano-1H-indol-7-yl)-1-methyl-4-imidazolesulfonamide
Compound 56
   N-(3-Chloro-1H-indol-7-yl)-6-[(2-hydroxyethyl)amino]-3-pyridinesulfonamide
Compound 57
   N-(3-Chloro-1H-indol-7-yl)-6-mercapto-3-pyridinesulfonamide
Compound 58
   7-(4-Chlorobenzenesulfonamido)-1H-indole-2-carboxylic acid
Compound 59
   N-(3-Chloro-1H-indol-7-yl)-6-cyclopropylamino-3-pyridinesulfonamide
Compound 60
   N-(3-Cyano-1H-indol-7-yl)-5-methyl-3-pyridinesulfonamide
Compound 61
   N-(3-Chloro-1H-indol-7-yl)-4-(N-methylsulfamoyl)-benzenesulfonamide
Compound 62
   N-(3-Chloro-1H-indol-7-yl)-4-[2-(methanesulfonamido)ethyl]-benzensulfonamide
Compound 63
   N-(3-Chloro-1H-indol-7-yl)-4-(sulfamoylmethyl)-benzensulfonamide
Compound 64
   N-(3-Chloro-1H-indol-7-yl)-4-thiocarbamoylbenzensulfonamide
Compound 65
   5-Bromo-N-(3-cyano-1H-indol-7-yl)-2-pyridinesulfonamide
Compound 66
   N-(3-Cyano-1H-indol-7-yl)-2-naphthalenesulfonamide
Compound 67
   N-(3-Acetyl-1H-indol-7-yl)-3-chlorobenzenesulfonamide
Compound 68
   4-Amino-N-(5-bromo-3-cyano-1H-indol-7-yl)benzenesulfonamide
Compound 69
   N-(3-Chloro-1H-indol-7-yl)-4-(N-ethylsulfamoyl)-benzenesulfonamide
Compound 70
   N-(3-Chloro-1H-indol-7-yl)-4-(ethanesulfonamido)-benzenesulfonamide
Compound 71
   N-(3-Chloro-1H-indol-7-yl)-6-[(2-cyanoethyl)amino]-3-pyridinesulfonamide
Compound 72
   N-(3-Chloro-1H-indol-7-yl)-4-(N-methylcarbamoyl)-benzenesulfonamide
Compound 73
   N-(3-Chloro-lH-indol-7-yl)-4-(methylsulfonylmethyl)-benzenesulfonamide
Compound 74
   N-(3-Chloro-1H-indol-7-yl)-4-(N,N-dimethylsulfamoyl)-benzenesulfonamide
Compound 75
   N-(3-Chloro-1H-indol-7-yl)-4-(1-pyrrolidinylcarbonyl)-benzenesulfonamide
Compound 76
   3-Chloro-N-(3-chloro-1H-indol-7-yl)-N-methylbenzenesulfonamide
Compound 77
   N-(3,4-Dichloro-1H-indol-7-yl)-4-(sulfamoylmethyl)-benzenesulfonamide
Compound 78
   N-(3-Cyano-1H-indol-7-yl)-4-[2-(methylsulfonyl)ethyl]-benzenesulfonamide
Compound 79
   N-(3-Chloro-1H-indol-7-yl)-4-(N-methylacetamido)-benzenesulfonamide
Compound 80
   N-(3-Chloro-1H-indol-7-yl)-6-hydroxy-3-pyridinesulfonamide
Compound 81
   N-(3-Chloro-1H-indol-7-yl)-4-[2-(N-methylmethanesulfonamido)ethyl]benzenesulfonamide
Compound 82
   N-(3-Chloro-1H-indol-7-yl)-4-(trifluoromethanesulfonamido)benzenesulfonamide
Compound 83
   N-(3-Chloro-1H-indol-7-yl)-4-[(N-methylmethanesulfonamido)methyl]benzenesulfonamide
Compound 84
   3-Chloro-N-(3-chloro-1H-pyrrolo[2,3-c]pyridin-7-yl)-benzenesulfonamide
Compound 85
   4-(3-Bromopropyl)-N-(3-chloro-1H-indol-7-yl)-benzenesulfonamide
Compound 86
   4-[N-(2-Bromoethyl)sulfamoyl]-N-(3-chloro-1H-indol-7-yl)-benzenesulfonamide
Compound 87
   N-(3-Chloro-1H-indol-7-yl)-4-[3-(1-imidazolyl)propyl]-benzenesulfonamide
Compound 88
   N-(3-Chloro-1H-indol-7-yl)-4-[N-(2-(2-pyridinyl)ethyl)-carbamoyl]benzenesulfonamide
Compound 89
   4-Amidino-N-(3-chloro-1H-indol-7-yl)benzenesulfonamide
Compound 90
   N-(3-Chloro-1H-indol-7-yl)-4-[N-[2-(1-imidazolyl)ethyl]-sulfamoyl]benzenesulfonamide
Compound 91
   3-(5-Bromonicotinamido)-N-(3-cyano-1H-indol-7-yl)-benzenesulfonamide
Compound 92
   N-(3-Chloro-1H-indol-7-yl)-4-[N-(2-thiazolyl)sulfamoyl]-benzenesulfonamide
Compound 93
   5-Chloro-N-(3-chloro-1H-indol-7-yl)-4-(5-methyl-3-pyridinesulfonamido)-2-thiophenesulfonamide
Compound 94
   3-Cyano-N-(3-cyano-4-methyl-1H-indol-7-yl)-benzenesulfonamide

The sulfonamide derivative or the sulfonic acid ester derivative represented by the general formula (I), a pharmacologically acceptable salt thereof, or a hydrate of the same (hereinafter, also referred as "the compound represented by the general formula (I)") may be manufactured by various methods. Among them, representative methods are described in JP 07-165708 A, JP 08-231505 A, and JP 2000-247949 A.

The compound represented by the general formula (I) has an excellent lymphocyte activation inhibition effect. Thus, the compound represented by the general formula (I) may be used as an active ingredient for a lymphocyte activation inhibitor. It may also be used as an active ingredient of a therapeutic agent for diseases in which inhibition of lymphocyte activation is effective to treatment thereof. Thus, according to the present invention, there is provided a method for inhibiting lymphocyte activation, comprising administering an effective amount of the compound represented by the general formula (I); and a method for treating a disease in which inhibition of lymphocyte activation is effective to treatment thereof, comprising administering an effective amount of the compound represented by the general formula (I). Also, according to the present invention, there is provided a use of the compound represented by the general formula (I) in manufacture of a lymphocyte activation inhibitor, and a use of the compound represented by the general formula (I) in manufacture of a therapeutic agent for a disease in which inhibition of lymphocyte activation is effective to treatment thereof.

The phrase "the compound represented by the general formula (I) as an active ingredient" includes a compound that generates the compound represented by the general formula (I) due to *in vivo* metabolism such as oxidation, reduction, and hydrolysis.

Similarly, the phrases "administering the compound represented by the general formula (I)" and "use of the compound represented by the general formula (I)" in the present invention also include administering and using a compound which generates the compound represented by the general formula (I) due to *in vivo* metabolism such as oxidation, reduction, and hydrolysis, respectively.

In the present invention, the word "treatment" also includes alleviating a symptom of a disease.

Examples of the disease in which inhibition of lymphocyte activation is effective to treatment thereof include autoimmune diseases. Examples of the autoimmune diseases include rheumatism, multiple sclerosis, neuro-autoimmune diseases (Guillain-Barre syndrome, neuro-Behcet's disease, etc.), type I (insulin-dependent) diabetes, systemic lupus erythematosus (SLE), inflammatory bowel diseases (IBD), and Sjogren's syndrome, etc.

The compound represented by the general formula (I) may be used in a preparation made by a general method. For example, it may be a composition with a carrier (if used for a medicine, a pharmacologically acceptable carrier).

The compound represented by the general formula (I), if used as a medicine, is administered orally or parenterally. The dose is different depending on degree of a symptom, age of a patient, sex, body weight, sensibility difference, administration method, administration timing, administration interval, medicinal characteristics, preparation, allegation, type, type of active ingredient, or the like. The dose is generally 10 to 6,000 mg, preferably about 50 to 4,000 mg, more preferably 100 to 3,000 mg per day for an adult, which was separately administered generally 1 to 3 time(s) in a day, but there is no particular limitation thereto.

In order to prepare an oral solid dosage form, a vehicle and optionally with a binder, disintegrator, lubricant, colorant, corrective, etc. are added to a base, and a mixture thereof is then treated in accordance with a conventional method to form tablets, coated tablets, granules, fine granules, powder, capsules, etc.

Examples of the vehicle include lactose, cornstarch, saccharose, glucose, sorbitol, crystalline cellulose, and silicon dioxide. Examples of the binder include polyvinyl alcohol, ethylcellulose, methylcellulose, acacia gum, hydroxypropylcellulose, and hydroxypropylmethylcellulose. Examples of the lubricant include magnesium stearate, talc, and silica. As the colorant, one permitted to add to a drug is used. Examples of the corrective include cocoa powder, menthol, aromatic acid, peppermint oil, borneol, and cinnamon powder. As a matter of course, those tablets and granules may optionally be coated appropriately with sugar, gelatin, etc.

In order to prepare an injection, a pH regulator, a buffer, a suspending agent, a solubilizer, a stabilizer, an isotonizing agent, a preservative, etc. are optionally added to a base, and a mixture thereof is then treated in accordance with a conventional method to form an injection for intravenous, hypodermic, or intramuscular injection. The injection may optionally be treated in accordance with a conventional method to form a freeze-dry product.

Examples of the suspending agent include methylcellulose, polysorbate 80, hydroxyethylcellulose, acacia gum, tragacanth powder, sodium carboxymethylcellulose, and polyoxyethylenesorbitan monolaurate.

Examples of the solubilizing agent include polyoxyethylene hardened castor oil, polysorbate 80, nicotinic acid amide, polyoxyethylenesorbitan monolaurate, macrogol, and castor oil fatty acid ethyl ester.

Examples of the stabilizer include sodium sulfite and sodium metasulfite. Examples of the preservative include methyl paraoxybenzoate, ethyl paraoxybenzoate, sorbic acid, phenol, cresol, and chlorocresol.

If the autoimmune disease is multiple sclerosis, the therapeutic agent for an autoimmune disease is preferred to further include a drug having a neuron-protecting effect.

The neuron-protecting effect means an effect of inhibiting a morbid change such as nerve axonal degeneration in multiple sclerosis by directly effecting to nerves.

Examples of the drug having a neuron-protecting effect include a glutamic acid antagonist, a sodium channel antagonist, a calcium channel antagonist, etc.

The compound represented by the general formula (I) and the drug having a neuron-protecting effect may be mixed or separately encased to be packed in a unit.

The compound represented by the general formula (I) and the drug having a neuron-protecting effect may be administered simultaneously or in order.

### Examples

The effect of the compound used in the present invention will now be shown with reference to pharmacological experimental examples.

### Pharmacological experimental example 1: Inhibition effect for lymphocyte activation

Immunizing a mouse with a partial peptide of a proteolipid protein (PLP), a central myelin protein, induces a chronic encephalomyelitis therein. Sampling lymphocytes from the spleen of the mouse and stimulating by an antigen in vitro allow the induction of antigen-specific activation and division of the lymphocyte. The effect of a drug to the reaction was examined to evaluate the inhibition effect of the drug for lymphocyte activation. Specifically, a female SJL/J mouse was immunized with an adjuvant containing a PLP partial peptide (PLP₁₃₉₋₁₅₁) under ether anesthesia. On the day of immunization and 2 days thereafter, deactivated *Bordetella pertussis* (one billion/mouse) was intravenously administered. On 7th or 8th day after the immunization, the mouse was killed by ether anesthesia and then the spleen thereof was extracted to isolate spleen lymphocytes. The lymphocytes were cultured with the PLP₁₃₉₋₁₅₁ in a medium containing 10% bovine fetal serum at 37°C under 5% CO₂ for 5 days. ³H-Thymidine (0.5 µCi/well) was added 16 hours before terminating the culture, and then radioactivity in the cells was measured to evaluate the inhibition effect of the compound for antigen-specific division of the lymphocytes, which was' referred as an IC50 value. The experimental results are shown in Table 1. The compound numbers are the same as those described in the previous list (hereinafter, the same).

**Table 1**

| Compound No. | IC50 (micro M) | Compond No. | IC50 (micro M) |
|---|---|---|---|
| 3 | 2.27 | 37 | 0.64 |
| 4 | 1.43 | 51 | 0.62 |
| 10 | 0.59 | 53 | 0.74 |
| 17 | 0.44 | 62 | 0.58 |
| 19 | 1.83 | 69 | 0.59 |
| 28 | 2.86 | 70 | 1.40 |
| 29 | 1.01 | 72 | 1.81 |
| 33 | 0.47 | 77 | 0.98 |
| 35 | 0.67 | 79 | 0.36 |
| 36 | 0.78 | 94 | 0.25 |

### Pharmacological experimental example 2: Effect on mouse collagen-induced arthritis

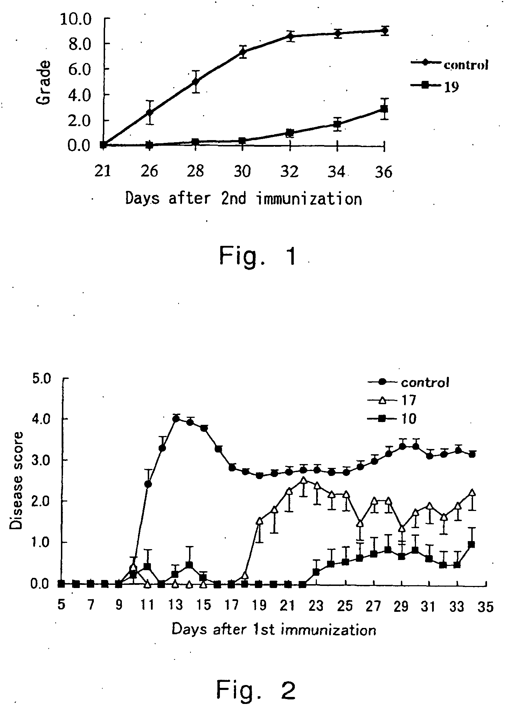

A DBA/1JN male mouse (7 weeks of age) was sensitized by intracutaneously injecting in the tail part thereof with 0.1 ml of an emulsion solution obtained by mixing a 0.3% bovine-derived type II collagen solution with the same amount of a Freund's complete adjuvant. After 3 weeks after initial sensitization, a booster was conducted in a similar way to cause arthritis. The subjective compound was made into a solution (5 mg/ml) according to a general method, and the day of the booster and 3 days thereafter, the solution was intravenously administered in an amount of 10 ml/kg (50 mg/kg). In experiments, 8 mice were used for each of a control group and a drug-administered group. The condition of arthritis was evaluated macroscopically on limbs and graded into 5 score levels (0; normal, 1; flare on a finger joint, 2; slight' edema on an entire leg, 3; edema on an entire leg, and 4; severe edema on an entire leg), to be shown as a total sum. As a result of the experiment using the Compound 19, the compound had an obvious inhibition effect for the crisis and development of arthritis. The effect lasted even after the second administration and the inhibition effect was recognized even 36 days after the initial sensitization, i.e., 12 days after administration (Fig. 1).

Note that, the mouse collagen-induced arthritis model described above is a model in which a lymphocyte activated by reacting with an immunized collagen recognizes an autotissue, particularly collagen in a joint tissue and causes an immunoreaction to induce arthritis, and is widely recognized as a model for evaluating a pharmacological. effect of a compound having a lymphocyte activation inhibition effect (e.g., Br. J. Rheumatol., 33, 798 (1994)). Pharmacological experimental example 3: Effect in an experimental encephalomyelitis model

100 µl of a Freund's complete adjuvant (Difco, containing 0.6 mg/ml of *M. tuberculosis* H37Ra) containing the PLP peptide (p139-151, Toray Research Center) was subcutaneously injected to a female SJL/J mouse in the back thereof under ether anesthesia for immunization (initial sensitization). Two days after, deactivated *Bordetella pertussis* was intravenously injected in an amount of about one billion per mouse. Seven days after, immunization was conducted again with the antigen. Over several weeks thereafter, abnormalities in limbs, a tail, and righting reflexes were graded into scored levels and recorded. The subjective compound was orally administered in an amount of 200 mg/kg from 5th day to 12th day after the initial sensitization. In experiments, 12 mice were used for each of a control group and a drug-administered group. As a result of experiments using the compounds shown in the Compounds 10 and 17 as subjective compounds, those compounds delayed expression of abnormality appeared in the mice, and alleviated the severity (Fig. 2).

Note that, the experimental encephalomyelitis model described above is a model in which a lymphocyte activated by reacting with an immunized PLP peptide causes an immunoreaction to induce encephalomyelitis, and is widely recognized as a model for evaluating a pharmacological effect of a compound having a lymphocyte activation inhibition effect (e.g., Agents and Actions, 27, 351-355 (1989); and Agents and Actions, 35, 79-84 (1992)).

As is apparent from the above experimental examples, the compound represented by the general formula (I) has an excellent lymphocyte activation inhibition effect. It is therefore considered to be useful as a therapeutic agent for autoimmune diseases, for treatment of rheumatism, multiple sclerosis, neuro-autoimmune diseases (Guillain-Barre syndrome, neuro-Behcet's disease, etc.), type I (insulin-dependent) diabetes, systemic lupus erythematosus (SLE), inflammatory bowel diseases (IBD), and Sjogren's syndrome, etc.

Moreover, even when the Compounds 10, 17, 51, and 94 were administered to mice successively for 15 days in amounts of 100 mg/kg and 200 mg/kg, respectively, the conditions of the mice were quite normal with dose-dependent weight increases and there was no observation showing toxicity. It is thus considered that the compound represented by the general formula (I) has low toxicity.

### Industrial Applicability

A lymphocyte activation inhibitor and a therapeutic agent for an autoimmune disease are provided.

## Claims

1. A lymphocyte activation inhibitor comprising, as an active ingredient, a sulfonamide derivative or sulfonic acid ester derivative represented by the following general formula (I): 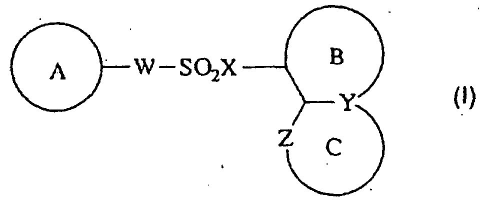 wherein the ring A represents a monocyclic or bicyclic aromatic ring which may be substituted,
the ring B represents a 6-membered unsaturated hydrocarbon ring or a 6-membered unsaturated heterocyclic ring containing one nitrogen atom as a heteroatom, each of which may be substituted,
the ring C represents a 5-membered heterocyclic ring containing one or two nitrogen atoms, which may be substituted,
W represents a single bond or -CH=CH-,
X represents -N(R¹)- or an oxygen atom,
Y represents a carbon atom or a nitrogen atom,
Z represents -N(R²)- or a nitrogen atom, and
R¹ and R² may be identical or different and each represents a hydrogen atom or a lower alkyl group, or a pharmacologically acceptable salt thereof, or a hydrate thereof.

2. The lymphocyte activation inhibitor according to claim 1, wherein W is a single bond.

3. The lymphocyte activation inhibitor according to claim 2, wherein X and Z are -NH-, and Y is a carbon atom.

4. The lymphocyte activation inhibitor according to any one of claims 1 to 3, wherein the ring B is benzene or pyridine, each of which may be substituted.

5. The lymphocyte activation inhibitor according to any one of claims 1 to 4, wherein the ring C is pyrrole which may be substituted.

6. The lymphocyte activation inhibitor according to claim 1, wherein the ring A is benzene or pyridine, each of which may be substituted; the ring B is benzene which may be substituted; the ring C is pyrrole which may be substituted; W is a single bond; and X and Z are -NH-.

7. A therapeutic agent for an autoimmune disease, comprising, as an active ingredient, a sulfonamide derivative or sulfonic acid ester derivative represented by the following general formula (I): 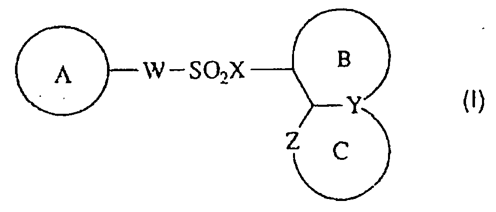 wherein the ring A represents a monocyclic or bicyclic aromatic ring which may be substituted,
the ring B represents a 6-membered unsaturated hydrocarbon ring or a 6-membered unsaturated heterocyclic ring containing one nitrogen atom as a heteroatom, each of which may be substituted,
the ring C represents a 5-membered heterocyclic ring containing one or two nitrogen atoms, which may be substituted,
W represents a single bond or -CH=CH-,
X represents -N(R¹)- or an oxygen atom,
Y represents a carbon atom or a nitrogen atom,
Z represents -N(R²)- or a nitrogen atom, and
R¹ and R² may be identical or different and each represents a hydrogen atom or a lower alkyl group, or a pharmacologically acceptable salt thereof, or a hydrate thereof.

8. The therapeutic agent according to claim 7, wherein W is a single bond.

9. The therapeutic agent according to claim 8, wherein X and Z are -NH-, and Y is a carbon atom.

10. The therapeutic agent according to any one of claims 7 to 9, wherein the ring B is benzene or pyridine, each of which may be substituted.

11. The therapeutic agent according to any one of claims 7 to 10, wherein the ring C is pyrrole which may be substituted.

12. The therapeutic agent according to claim 7, wherein the ring A is benzene or pyridine, each of which may be substituted; the ring B is benzene which may be substituted; the ring C is pyrrole which may be substituted; W is a single bond; and X and Z are -NH-.

13. The therapeutic agent according to any one of claims 7 to 12, wherein the autoimmune disease is a disease selected from the group consisting of cellular autoimmune diseases, rheumatism, multiple sclerosis, neuro-autoimmune diseases, type I (insulin-dependent) diabetes, systemic lupus erythematosus, inflammatory bowel diseases, and Sjogren's syndrome.

14. The therapeutic agent according to claim 13, wherein the autoimmune disease is multiple sclerosis, and the agent further comprises a drug having a neuron-protecting effect.
